# DEMANDE DE BREVET EUROPEEN

(11) **EP 3 178 397 A1**
(43) Date de publication de la demande: **14.06.2017**
(21) Numéro de dépôt: 16200998.9
(22) Date de dépôt: 28.11.2016
(51) Int. Cl.: A61B 5/11, A61B 5/00

(54) **SYSTEME ET PROCEDE DE DETECTION DU PORT PAR UN PATIENT D'UN APPAREIL DE DECHARGE DE PLAIES DES PIEDS**

(30) Priorité: 07.12.2015 FR 1561949
(71) Demandeur: Creative Specific Software, 83700 Saint-Raphael (FR)
(72) Inventeur: LARBI, Ramzi, 06600 Antibes (FR)
(74) Mandataire: Novagraaf Technologies

(57) **Abrégé**

La présente invention se rapporte à un système (1) et à un procédé de détection du port par un patient d'un appareil (4) de décharge de plaies des pieds. Le système (1) comprend un dispositif électronique de géolocalisation (10) et un équipement (12) de traitement de données relié au dispositif électronique de géolocalisation (10) via un réseau de communication (14), le dispositif électronique de géolocalisation (10) étant adapté pour être solidarisé à l'appareil de décharge (4) et comportant des moyens d'alimentation électrique (26), des moyens (20) d'acquisition de données de géolocalisation et des moyens (22) d'émission de données de géolocalisation sur le réseau de communication (14) à destination de l'équipement (12) de traitement de données, les moyens (22) d'émission de données étant reliés aux moyens d'acquisition (20), l'équipement (12) de traitement de données étant propre, suite à la réception de données de géolocalisation émises par le dispositif électronique de géolocalisation (10), à détecter un mouvement de l'appareil (4) de décharge de plaies des pieds et à en déduire le port par le patient (2) dudit appareil de décharge (4).

## Description

### Domaine technique

La présente invention se rapporte à un système, un produit programme d'ordinateur et un procédé de détection du port par un patient d'un appareil de décharge de plaies des pieds.

### Etat de la technique

Le pied est une cible privilégiée des complications du diabète. En effet, les plaies du pied chez le diabétique constituent un véritable problème de santé publique du fait de l'épidémiologie de cette affection, de la sévérité des complications, du coût pour la société et enfin de la précarité de la population concernée. Or, le nombre croissant de patients diabétiques va retentir sur les complications du diabète et plus particulièrement sur la survenue du mal perforant plantaire.

Pour résoudre le problème de la guérison ou de la prévention de l'apparition de plaies aux pieds chez les patients diabétiques, il est connu d'utiliser des appareils de décharge de plaies des pieds. La décharge d'une plaie peut se définir comme toute mesure visant à éliminer les points anormaux de pression s'exerçant sur la plaie pour en faciliter la cicatrisation ou en prévenir la récidive.

Un appareil de décharge de plaies des pieds peut par exemple se présenter sous la forme d'une chaussure médicale spécialement adaptée pour la décharge des plaies. Le principe d'un tel appareil de décharge est de permettre au patient le maintien d'une autonomie avec une activité physique indispensable au traitement de l'artériopathie et à l'équilibre du diabète tout en évitant l'hyper appui sur la plaie. La décharge est le traitement qui donne les meilleurs résultats en termes de taux et de durée de cicatrisation. Elle diminue le risque d'infection et d'amputation. Néanmoins, pour être efficace, la décharge doit être réalisée 24 heures sur 24 et 7 jours sur 7. Abandonner ce traitement est donc très risqué et le non suivi par le patient du traitement médical par décharge doit être dépisté au plus tôt par le praticien suivant le patient, afin d'ajuster éventuellement la stratégie thérapeutique de prise en charge du patient.

Or, de tels appareils de décharge sont généralement peu pratiques à utiliser et relativement inconfortables et/ou inesthétiques. De ce fait un nombre élevé de patients ne respectent pas la prescription médicale du port permanent de l'appareil de décharge, et n'informent pas leur praticien de cette inobservance du traitement. Un des problèmes rencontrés par les praticiens est donc celui de la difficulté de quantifier la durée de port de l'appareil de décharge par le patient.

Pour résoudre ce problème, on connait des systèmes de détection du port par un patient d'un appareil de décharge de plaies des pieds. Un tel système est par exemple décrit dans le document US 2009/0209830 A1. Le système de détection comprend un dispositif électronique par exemple agencé dans la semelle d'une chaussure de décharge des plaies, et un équipement de traitement de données relié au dispositif électronique via un réseau de communication. Le dispositif électronique comporte un capteur de pression et des moyens d'émission de données de mesure de pression, sur le réseau de communication à destination de l'équipement de traitement de données. Ainsi, le port ou non de l'appareil de décharge par le patient peut être détecté par l'équipement de traitement de données, par comparaison des valeurs de pression mesurées à un seuil prédéterminé. Si une valeur de pression mesurée est inférieure au seuil, cela signifie que le patient ne porte pas la chaussure de décharge.

Toutefois, un tel système de détection présente une faible précision du fait du mode de détection par comparaison de valeurs de pression mesurées, notamment pour des valeurs de pression s'établissant autour du seuil prédéterminé. En outre, un tel système de détection ne permet pas de déterminer la distance parcourue par le patient muni de son appareil de décharge des plaies, ni de quantifier avec précision la durée de port de l'appareil de décharge par le patient.

Il existe donc un réel besoin d'un système et d'un procédé de détection du port par un patient d'un appareil de décharge de plaies des pieds palliant ces défauts, inconvénients et obstacles de l'art antérieur, en particulier d'un système simple et fiable présentant une précision de détection améliorée, tout en permettant de déterminer la distance parcourue par le patient muni de l'appareil de décharge ainsi que la durée du parcours.

### Description de l'invention

Pour résoudre un ou plusieurs des inconvénients de l'art antérieur, selon un aspect de l'invention, un système de détection du port par un patient d'un appareil de décharge de plaies des pieds comprend un dispositif électronique de géolocalisation et un équipement de traitement de données relié au dispositif électronique de géolocalisation via un réseau de communication, le dispositif électronique de géolocalisation étant adapté pour être solidarisé à l'appareil de décharge et comportant des moyens d'alimentation électrique, des moyens d'acquisition de données de géolocalisation et des moyens d'émission de données de géolocalisation sur le réseau de communication à destination de l'équipement de traitement de données, les moyens d'émission de données étant reliés aux moyens d'acquisition, l'équipement de traitement de données étant propre, suite à la réception de données de géolocalisation émises par le dispositif électronique de géolocalisation, à détecter un mouvement de l'appareil de décharge de plaies des pieds et à en déduire le port par le patient dudit appareil de décharge.

Grâce à la présence au sein du dispositif électronique de moyens d'acquisition de données de géolocalisation, les données reçues par l'équipement de traitement de données sont des données de géolocalisation, émises par le dispositif, par exemple de manière périodique. La détection du port par le patient de l'appareil de décharge est effectuée par l'équipement de traitement de données, soit par détection d'un changement de valeur entre deux valeurs consécutives des données de géolocalisation reçues, soit via la simple réception des données de géolocalisation. La précision de la détection est ainsi dans tous les cas améliorée. En outre, puisque des données de position du dispositif électronique sont reçues séquentiellement par l'équipement de traitement de données, le système de détection selon l'invention permet avantageusement de déterminer la distance parcourue par le patient muni de l'appareil de décharge, ainsi que de déterminer indirectement la durée du parcours.

Selon des modes de réalisation particuliers utilisables seuls ou en combinaison :
- le dispositif électronique de géolocalisation comprend en outre un capteur de mouvement relié aux moyens d'émission de données de géolocalisation, les moyens d'émission de données de géolocalisation étant configurés pour transmettre des données de géolocalisation à l'équipement de traitement de données lorsqu'un mouvement du dispositif est détecté par le capteur de mouvement ;
- le système comprend en outre un deuxième dispositif électronique de géolocalisation adapté pour être fixé directement sur le corps du patient, le deuxième dispositif de géolocalisation étant relié à l'équipement de traitement de données via le réseau de communication et comprenant des deuxièmes moyens d'alimentation électrique, des deuxièmes moyens d'acquisition de données de géolocalisation et des deuxièmes moyens d'émission de données de géolocalisation sur le réseau de communication à destination de l'équipement de traitement de données, les deuxièmes moyens d'émission de données étant reliés aux deuxièmes moyens d'acquisition ;
- le deuxième dispositif électronique de géolocalisation comprend en outre un deuxième capteur de mouvement relié aux deuxièmes moyens d'émission de données de géolocalisation, les deuxièmes moyens d'émission de données de géolocalisation étant configurés pour transmettre des données de géolocalisation à l'équipement de traitement de données lorsqu'un mouvement du dispositif est détecté par le deuxième capteur de mouvement ;
- l'un au moins des dispositifs de géolocalisation comprend une balise de traçage GPS à déclenchement instantané sur mouvement du dispositif ;
- l'équipement de traitement de données est propre, suite à la réception des données de géolocalisation émises par les dispositifs électroniques de géolocalisation, à calculer un taux de décharge associé au port par le patient de l'appareil de décharge de plaies des pieds ;
- le système comprend en outre un dispositif électronique de communication adapté pour être fixé directement sur le corps du patient, le dispositif électronique de communication comprenant des moyens d'alimentation électrique et un émetteur - récepteur de signaux radioélectriques ;
- le dispositif électronique de communication comprend en outre un capteur de mouvement relié à l'émetteur - récepteur de signaux radioélectriques, l'émetteur - récepteur étant configuré pour émettre des signaux radioélectriques lorsqu'un mouvement du dispositif de communication est détecté par le capteur de mouvement ;
- le dispositif électronique de géolocalisation comprend en outre un microcontrôleur programmable relié aux moyens d'émission de données, et un émetteur - récepteur de signaux radioélectriques relié au microcontrôleur programmable, le microcontrôleur étant programmé pour transmettre un signal de commande d'émission de données de géolocalisation aux moyens d'émission de données, accompagné d'une première notification si une liaison radioélectrique a été établie entre l'émetteur - récepteur et le dispositif électronique de communication ; et pour transmettre un signal de commande d'émission de données de géolocalisation aux moyens d'émission de données, accompagné d'une deuxième notification, différente de la première notification, si aucune liaison radioélectrique n'a été établie entre l'émetteur - récepteur et le dispositif électronique de communication ;
- l'équipement de traitement de données est propre, suite à la réception des données de géolocalisation émises par le dispositif électronique de géolocalisation et de la première ou de la deuxième notification, à calculer un taux de décharge associé au port par le patient de l'appareil de décharge de plaies des pieds ;
- l'équipement de traitement de données comporte un serveur, le serveur comprenant un processeur et une mémoire reliée au processeur, la mémoire stockant une application, l'application étant propre, lorsqu'elle est mise en oeuvre par le processeur, à détecter le mouvement de l'appareil de décharge de plaies des pieds et à en déduire le port par le patient dudit appareil de décharge, suite à la réception des données de géolocalisation émises par le dispositif électronique de géolocalisation.

Selon un deuxième aspect de l'invention, un procédé de détection du port par un patient d'un appareil de décharge de plaies des pieds est mis en oeuvre par un système de détection comprenant un dispositif électronique de géolocalisation et un équipement de traitement de données relié au dispositif électronique de géolocalisation via un réseau de communication, le dispositif électronique de géolocalisation étant solidarisé à l'appareil de décharge et comportant des moyens d'alimentation électrique, des moyens d'acquisition de données de géolocalisation, et des moyens d'émission de données de géolocalisation sur le réseau de communication à destination de l'équipement de traitement de données, les moyens d'émission de données étant reliés aux moyens d'acquisition, le procédé comprenant les étapes suivantes :
- acquisition de données de géolocalisation par les moyens d'acquisition de données ;
- émission des données de géolocalisation acquises sur le réseau de communication, à destination de l'équipement de traitement de données ;
- détection par l'équipement de traitement de données, à partir des données de géolocalisation reçues, d'un mouvement de l'appareil de décharge de plaies des pieds, et, par suite, du port par le patient dudit appareil de décharge.

Selon un mode de réalisation particulier, le système de détection comprend en outre un deuxième dispositif électronique de géolocalisation fixé directement sur le corps du patient, le deuxième dispositif de géolocalisation étant relié à l'équipement de traitement de données via le réseau de communication et comprenant des deuxièmes moyens d'alimentation électrique, des deuxièmes moyens d'acquisition de données de géolocalisation et des deuxièmes moyens d'émission de données de géolocalisation sur le réseau de communication à destination de l'équipement de traitement de donnée, le procédé comprenant en outre les étapes suivantes :
- acquisition de données de géolocalisation par les deuxièmes moyens d'acquisition de données ;
- émission des données de géolocalisation acquises par les deuxièmes moyens d'acquisition sur le réseau de communication, à destination de l'équipement de traitement de données ;
- calcul, par l'équipement de traitement de données, d'un taux de décharge associé au port par le patient de l'appareil de décharge de plaies des pieds, que l'équipement de traitement de données ait détecté ou non le port par le patient de l'appareil de décharge de plaies des pieds.

Selon un autre mode de réalisation particulier, le système de détection comprend en outre un dispositif électronique de communication fixé directement sur le corps du patient, le dispositif électronique de communication comprenant des moyens d'alimentation électrique et un émetteur - récepteur de signaux radioélectriques, le dispositif électronique de géolocalisation comprenant en outre un microcontrôleur programmable relié aux moyens d'émission de données, et un émetteur - récepteur de signaux radioélectriques relié au microcontrôleur programmable, le procédé comprenant en outre les étapes suivantes :
- détermination, par le microcontrôleur programmable, si une liaison radioélectrique a été établie entre l'émetteur-récepteur du dispositif électronique de géolocalisation et l'émetteur-récepteur du dispositif électronique de communication ;
- transmission, par le microcontrôleur programmable, d'un signal de commande d'émission de données de géolocalisation aux moyens d'émission de données, accompagné d'une première notification si une liaison radioélectrique a été établie entre le dispositif électronique de géolocalisation et le dispositif électronique de communication ; ou d'une deuxième notification, différente de la première notification, si aucune liaison radioélectrique n'a été établie entre le dispositif électronique de géolocalisation et le dispositif électronique de communication ;
- calcul, par l'équipement de traitement de données, d'un taux de décharge associé au port par le patient de l'appareil de décharge de plaies des pieds, que l'équipement de traitement de données ait détecté ou non le port par le patient de l'appareil de décharge de plaies des pieds ;
et, lors de l'étape d'émission des données de géolocalisation acquises, lesdites données de géolocalisation émises sont accompagnées de la première ou de la deuxième notification.

Selon un troisième aspect de l'invention, un produit programme d'ordinateur téléchargeable depuis un réseau de communication et/ou enregistré sur un support lisible par ordinateur et/ou exécutable par un processeur, comprend des instructions de programme, lesdites instructions de programme formant l'application du serveur de l'équipement de traitement de données tel que décrit ci-dessus, lesdites instructions de programme étant adaptées pour mettre en oeuvre les étapes de détection d'un mouvement de l'appareil de décharge de plaies des pieds, et, par suite, du port par le patient dudit appareil de décharge, du procédé tel que décrit ci-dessus lorsque le produit programme est exécuté sur ledit serveur.

D'autres avantages pourront encore apparaître à l'homme du métier à la lecture des exemples ci-dessous, illustrés par les figures annexées, donnés à titre illustratif.

### Brève description des figures

- La figure 1 est une représentation schématique d'un système de détection du port par un patient d'un appareil de décharge de plaies des pieds selon un premier mode de réalisation de l'invention ;
- La figure 2 représente l'architecture fonctionnelle du système de détection de la figure 1 ;
- La figure 3 est un organigramme représentant un procédé de détection du port par un patient d'un appareil de décharge de plaies des pieds selon l'invention, tel que mis en oeuvre par le système de la figure 2 ;
- La figure 4 est une vue analogue à celle de la figure 2 d'un système de détection du port par un patient d'un appareil de décharge de plaies des pieds selon un deuxième mode de réalisation.

### Modes de réalisation

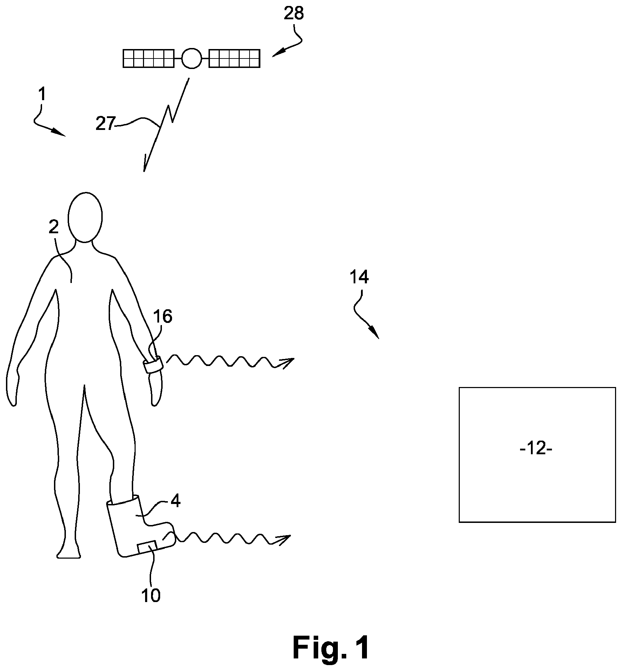

Sur la figure 1 est représenté un système 1 de détection du port par un patient 2 d'un appareil 4 de décharge de plaies des pieds selon un premier mode de réalisation de l'invention. L'appareil de décharge 4 peut être un appareil amovible ou inamovible. Dans l'exemple de réalisation particulier de la figure 1, l'appareil de décharge 4 est une chaussure médicale spécialement adaptée pour la décharge des plaies des pieds.

Le système de détection 1 comprend un dispositif électronique de géolocalisation 10 et un équipement 12 de traitement de données relié au dispositif électronique 10 via un réseau de communication 14. Dans l'exemple de réalisation particulier de la figure 1, le système de détection 1 comprend un premier dispositif électronique de géolocalisation 10 et un deuxième dispositif électronique de géolocalisation 16.

Le premier dispositif électronique de géolocalisation 10 est adapté pour être solidarisé à l'appareil de décharge 4. Dans l'exemple de réalisation particulier de la figure 1, le premier dispositif électronique de géolocalisation 10 est adapté pour être agencé au sein d'une chaussure médicale spécialement adaptée pour la décharge des plaies des pieds, par exemple, mais non exclusivement, au sein d'une semelle de cette chaussure. En variante non représentée, le premier dispositif électronique de géolocalisation 10 est adapté pour être fixé sur la chaussure médicale.

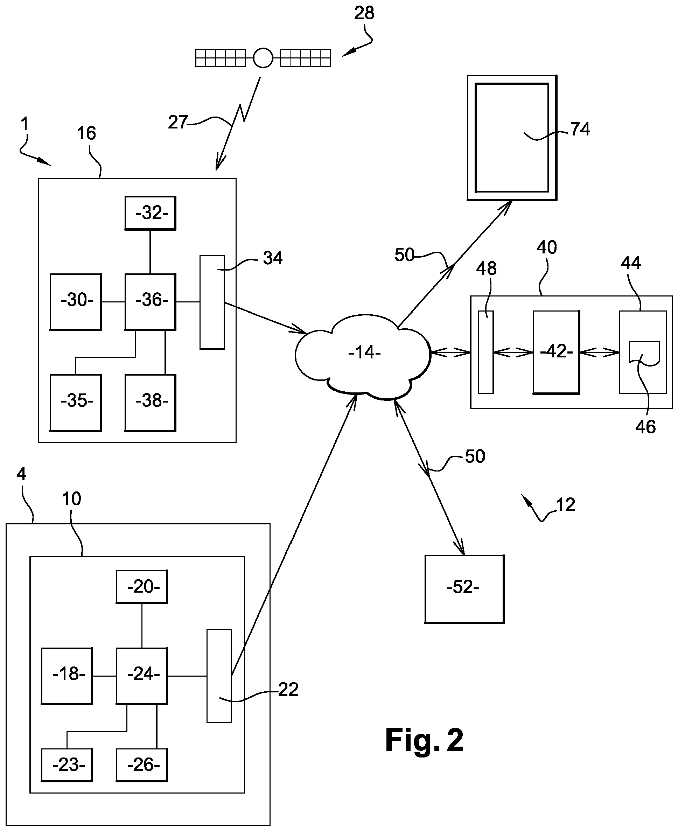

Comme illustré sur la figure 2, le premier dispositif électronique de géolocalisation 10 comprend des moyens d'alimentation électrique 26, des moyens 20 d'acquisition de données de géolocalisation, et des moyens 22 d'émission de données de géolocalisation sur le réseau de communication 14, à destination de l'équipement 12 de traitement de données. De préférence, comme illustré sur la figure 2, le premier dispositif électronique de géolocalisation 10 comprend en outre un capteur de mouvement 18, un podomètre 23 et un microcontrôleur programmable 24. En variante, le microcontrôleur programmable 24 peut être remplacé par tout type de moyen de calcul et de traitement de données, tel que par exemple un processeur ou un microprocesseur.

Le premier dispositif électronique de géolocalisation 10 est par exemple formé d'un boîtier de protection au sein duquel est agencée une balise de traçage GPS (de l'anglais Global Positioning System), de préférence une balise de traçage GPS à déclenchement instantané sur mouvement du dispositif. Dans le cas particulier où le premier dispositif de géolocalisation 10 est adapté pour être agencé au sein de l'appareil 4 de décharge de plaies des pieds, le premier dispositif électronique de géolocalisation 10 présente des dimensions compatibles avec un tel agencement. Le premier dispositif électronique de géolocalisation 10 présente en particulier des dimensions compatibles avec un agencement au sein d'une chaussure médicale, notamment avec un agencement au sein d'une semelle d'une telle chaussure, de sorte à ne pas nuire au confort du patient portant la chaussure, ni à gêner ou entraver le mouvement de marche du patient. L'épaisseur du premier dispositif électronique de géolocalisation 10 est par exemple de l'ordre de quelques millimètres.

Les moyens d'alimentation électrique 26 sont par exemple aptes à fournir une tension électrique nominale sensiblement égale à 12 V. Les moyens d'alimentation électrique 26 sont par exemple formés d'une batterie lithium, notamment une batterie lithium rechargeable via un connecteur de type micro USB (de l'anglais Universal Sérial Bus). Les moyens d'alimentation électrique 26 sont par exemple aptes à fournir une autonomie électrique au premier dispositif électronique de géolocalisation 10 de l'ordre de plusieurs mois.

Les moyens 20 d'acquisition de données de géolocalisation sont configurés pour déclencher périodiquement une acquisition de données de géolocalisation. Plus précisément, dans l'exemple de réalisation préférentiel illustré sur la figure 2, le microcontrôleur programmable 24 est programmé pour transmettre périodiquement un signal de commande d'acquisition de données de géolocalisation aux moyens 20 d'acquisition de données de géolocalisation. Les moyens 20 d'acquisition de données de géolocalisation comprennent par exemple une antenne GPS, apte à recevoir et acquérir des signaux GPS 27 émis par un satellite de positionnement GPS 28.

Les moyens 22 d'émission de données de géolocalisation sur le réseau de communication 14 sont reliés aux moyens 20 d'acquisition de données de géolocalisation. Les moyens 22 d'émission de données comprennent par exemple une antenne conforme à la norme GSM (de l'anglais Global System for Mobile Communication).

Le capteur de mouvement 18 est relié aux moyens 22 d'émission de données de géolocalisation. Dans l'exemple de réalisation préférentiel illustré sur la figure 2, le capteur de mouvement 18 est connecté au microcontrôleur programmable 24, ce dernier étant également connecté aux moyens 22 d'émission de données de géolocalisation. Le capteur de mouvement 18 est par exemple un accéléromètre linéaire trois axes, apte à délivrer un signal dès qu'une valeur d'accélération non nulle est mesurée. En variante, le capteur de mouvement 18 est un capteur de vitesse instantanée. Dans l'exemple de réalisation préférentiel selon lequel le premier dispositif électronique de géolocalisation 10 comprend un capteur de mouvement 18, les moyens 22 d'émission de données de géolocalisation sont configurés pour transmettre des données de géolocalisation à l'équipement 12 de traitement de données lorsqu'un mouvement du dispositif 10 est détecté par le capteur de mouvement 18. Cet exemple de réalisation préférentiel permet avantageusement d'économiser de l'énergie électrique au sein des moyens d'alimentation électrique 26, les moyens d'émission 22 n'émettant pas des données sur une base périodique mais seulement si un mouvement du dispositif 10 a été détecté par le capteur de mouvement 18. En outre, cet exemple de réalisation préférentiel permet également d'améliorer la précision de la détection. Le temps d'acquisition et d'émission de données de géolocalisation par le premier dispositif 10, à partir de l'instant où un mouvement du premier dispositif 10 est détecté par le capteur de mouvement 18, est de préférence inférieur à 1 seconde.

Le podomètre 23 est connecté au microcontrôleur programmable 24 et est apte à fournir des données de mesure du nombre de pas du patient 2.

Le microcontrôleur programmable 24 est relié au capteur de mouvement 18, aux moyens 20 d'acquisition de données de géolocalisation, aux moyens 22 d'émission de données de géolocalisation, au podomètre 23 et aux moyens d'alimentation électrique 26. Le microcontrôleur programmable 24 est par exemple programmé pour transmettre périodiquement un signal de commande d'émission de données aux moyens 22 d'émission de données. Par exemple, la période de transmission du signal de commande d'émission de données peut être programmée pour être sensiblement égale à 10 minutes. En variante, dans l'exemple de réalisation préférentiel selon lequel le premier dispositif électronique de géolocalisation 10 comprend un capteur de mouvement 18, le microcontrôleur programmable 24 est programmé pour transmettre un signal de commande d'émission de données aux moyens 22 d'émission de données, lorsqu'un mouvement du dispositif 10 est détecté par le capteur de mouvement 18. Outre l'économie d'énergie électrique au sein des moyens d'alimentation électrique 26 précédemment évoquée, ceci permet de réduire le nombre de transmissions de données sur le réseau 14 par le dispositif électronique 10, et permet ainsi de réduire avantageusement la consommation de ressources informatiques nécessaires à la détection du port par le patient 2 de l'appareil 4.

Dans l'exemple de réalisation selon lequel le premier dispositif électronique de géolocalisation 10 comprend un podomètre 23, les données émises par les moyens 22 d'émission de données comprennent, outre les données de géolocalisation, les données de mesure du nombre de pas du patient 2 fournies par le podomètre 23.

Le deuxième dispositif électronique de géolocalisation 16 est relié à l'équipement 12 de traitement de données via le réseau de communication 14. Le deuxième dispositif électronique de géolocalisation 16 est adapté pour être fixé directement sur le corps du patient 2, par exemple sur un poignet de ce dernier, comme illustré sur la figure 1. Dans ce cas, le deuxième dispositif électronique de géolocalisation 16 se présente par exemple sous la forme d'un bracelet électronique de géolocalisation. Le bracelet électronique comporte par exemple une balise de traçage GPS, de préférence une balise de traçage GPS à déclenchement instantané sur mouvement du dispositif. Le deuxième dispositif électronique de géolocalisation 16 présente des dimensions compatibles avec un port sur un membre du patient, typiquement un poignet, qui ne soit pas gênant pour le patient durant son mouvement de marche. L'épaisseur de la balise de traçage GPS au sein du deuxième dispositif électronique de géolocalisation 16 est par exemple de l'ordre de quelques millimètres.

Le deuxième dispositif électronique de géolocalisation 16 comprend des composants analogues à ceux du premier dispositif électronique de géolocalisation 10. Ainsi, le deuxième dispositif électronique de géolocalisation 16 comprend des moyens d'alimentation électrique 38, des moyens 32 d'acquisition de données de géolocalisation, et des moyens 34 d'émission de données de géolocalisation sur le réseau de communication 14, à destination de l'équipement 12 de traitement de données. De préférence, comme illustré sur la figure 2, le deuxième dispositif électronique de géolocalisation 16 comprend en outre un capteur de mouvement 30, un podomètre 35 et un microcontrôleur programmable 36. En variante, le microcontrôleur programmable 36 peut être remplacé par tout type de moyen de calcul et de traitement de données, tel que par exemple un processeur ou un microprocesseur. Les structures et fonctionnalités des composants 30, 32, 34, 35, 36, 38 sont identiques à celles des composants homologues 18, 20, 22, 23, 24, 26 du premier dispositif électronique de géolocalisation 10, et ne seront donc pas décrites en détail. Par exemple, les moyens 32 d'acquisition de données de géolocalisation sont reliés aux moyens 34 d'émission de données et sont configurés pour déclencher périodiquement une acquisition de données.

L'équipement 12 de traitement de données est propre, suite à la réception de données de géolocalisation émises par le premier dispositif de géolocalisation 10, à détecter un mouvement de l'appareil 4 de décharge de plaies des pieds et à en déduire le port par le patient 2 de l'appareil de décharge 4. Plus précisément, comme illustré sur la figure 2, l'équipement 12 de traitement de données comporte un serveur 40. Le serveur 40 comprend un processeur 42 et une mémoire 44 reliée au processeur 42. La mémoire stocke une application 46 propre, lorsqu'elle est mise en oeuvre par le processeur 42, à détecter un mouvement de l'appareil 4 de décharge de plaies des pieds et à en déduire le port par le patient 2 de l'appareil de décharge 4, suite à la réception de données de géolocalisation émises par le premier dispositif de géolocalisation 10. L'application 46 est également propre, lorsqu'elle est mise en oeuvre par le processeur 42, à déterminer la distance parcourue par le patient 2 muni de l'appareil de décharge 4 ou encore la durée de port de l'appareil de décharge 4. Dans l'exemple de réalisation selon lequel le premier dispositif électronique de géolocalisation 10 comprend un podomètre 23, cette détermination par l'application 46 de la distance parcourue par le patient 2 muni de l'appareil de décharge 4 est effectuée par moyennage entre une distance estimée à partir des données de géolocalisation reçues, et une distance estimée à partir des données de mesure du nombre de pas du patient 2.

Le serveur 40 comporte également des moyens 48 d'envoi de messages 50 sur le réseau de communication 14, reliés au processeur 42.

De préférence, le serveur 40 est un serveur web et héberge une interface d'accès web, une telle interface n'étant pas représentée sur les figures pour des raisons de clarté. De préférence encore, le serveur 40 de l'équipement 12 de traitement de données est propre, via la mise en oeuvre de l'application 46 par le processeur 42, à générer périodiquement des tableaux et/ou des listes de suivi de l'activité du patient 2, et/ou des données d'actigraphie - actimétrie relatives au patient 2, et/ou des données comparatives de l'activité du patient 2, et/ou des données indicatives de l'état des balises de traçage GPS ou des moyens d'alimentation électrique 26, 38.

Dans le mode de réalisation particulier illustré sur la figure 2, l'équipement 12 de traitement de données comporte en outre une station de surveillance 52. La station de surveillance 52 comporte par exemple des moyens d'affichage et/ou des moyens d'envoi de messages sur le réseau de communication 14.

Dans l'exemple de réalisation particulier des figures 1 et 2 selon lequel le système de détection 1 comprend un deuxième dispositif de géolocalisation 16, l'équipement 12 de traitement de données est propre, suite à la réception des données de géolocalisation émises par le deuxième dispositif électronique de géolocalisation 16 et éventuellement par le premier dispositif électronique de géolocalisation 10, à calculer un taux de décharge associé au port par le patient 2 de l'appareil de décharge 4. Plus précisément, selon cet exemple de réalisation particulier, l'application 46 est propre, lorsqu'elle est mise en oeuvre par le processeur 42, à calculer le taux de décharge, suite à la réception des données de géolocalisation émises par le deuxième dispositif 16 et éventuellement par le premier dispositif 10. Le taux de décharge associé au port par le patient 2 de l'appareil de décharge 4 est défini comme le rapport entre la durée pendant laquelle le patient se déplace sans l'appareil de décharge 4 et la durée pendant laquelle il se déplace avec l'appareil de décharge 4. La durée pendant laquelle le patient se déplace avec l'appareil de décharge 4 peut être déduite de la réception des données de géolocalisation émises par le premier dispositif de géolocalisation 10, comme décrit ci-dessus.

La durée pendant laquelle le patient se déplace sans l'appareil de décharge 4 peut par exemple être déduite de la réception de données de géolocalisation émises par le deuxième dispositif de géolocalisation 16 et présentant des valeurs successives distinctes, et de la réception de données de géolocalisation émises par le premier dispositif de géolocalisation 10 et présentant des valeurs successives constantes, dans l'exemple de réalisation selon lequel les premier et deuxième dispositifs de géolocalisation 10, 16 ne comportent pas de capteur de mouvement. En effet, selon cet exemple de réalisation, quand le patient 2 se déplace sans l'appareil de décharge 4, le deuxième dispositif de géolocalisation 16 émet des données de géolocalisation présentant des valeurs successives distinctes, tandis que le premier dispositif de géolocalisation 10 émet des données de géolocalisation présentant des valeurs successives constantes. La durée pendant laquelle le patient se déplace sans l'appareil de décharge 4 peut également être déduite de la réception des données de géolocalisation émises par le deuxième dispositif de géolocalisation 16, et de la non réception de données de géolocalisation de la part du premier dispositif de géolocalisation 10, dans l'exemple de réalisation selon lequel les premier et deuxième dispositifs de géolocalisation 10, 16 comprennent chacun un capteur de mouvement 18, 30. En effet, selon cet exemple de réalisation, quand le patient 2 se déplace sans l'appareil de décharge 4, le deuxième dispositif de géolocalisation 16 émet des données de géolocalisation sur le réseau de communication 14, tandis que le premier dispositif de géolocalisation 10 n'en émet pas.

Le calcul d'un tel taux de décharge est particulièrement utile à un praticien suivant le patient 2. En effet, le praticien peut alors avantageusement évaluer l'observance à la décharge de son patient. En particulier, grâce au calcul de ce taux de décharge, le praticien peut discriminer entre les patients non observant et les patients observant correctement leur traitement mais ayant une évolution défavorable malgré une décharge correcte. Les premiers, qui ne portent pas systématiquement leur appareil de décharge 4 lorsqu'ils marchent, relèveront d'une éducation renforcée pour le suivi correct de leur traitement. Les seconds, qui portent systématiquement leur appareil de décharge 4 lorsqu'ils marchent, nécessiteront des thérapeutiques complémentaires. Les caractéristiques relatives à la présence d'un deuxième dispositif de géolocalisation 16 permettent ainsi d'améliorer encore la précision de la détection et de la détermination de la durée de port de l'appareil de décharge par le patient, et de permettre à un praticien d'affiner et d'améliorer la stratégie thérapeutique de prise en charge de ses patients.

Le réseau de communication 14 est muni d'une infrastructure de communication privée ou étendue permettant la connexion, ou l'accès, à des équipements de communication de type serveurs et/ou bases de données et/ou dispositifs électroniques de communication. De manière classique, l'infrastructure de communication forme un réseau sans fil, ou un réseau comprenant une portion sans fil et une portion filaire. Selon un exemple de réalisation particulier, le réseau de communication 14 est conçu comme un réseau cellulaire de type GSM (de l'anglais Global System for Mobile Communication), ou GPRS (de l'anglais General Packet Radio Service) ou encore UMTS (de l'anglais Universal Mobile Telecommunications System). En variante, le réseau de communication 14 peut être conçu comme un réseau de type Internet, comportant par exemple une portion de réseau conforme à la norme GSM, GPRS ou UMTS.

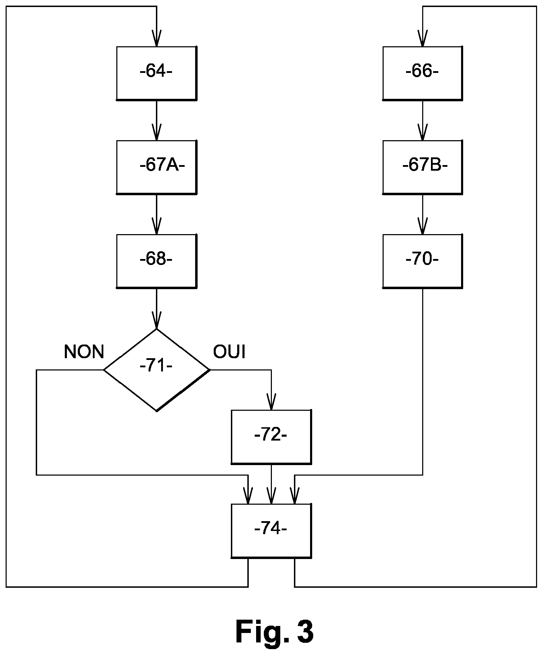

Le procédé de détection du port par le patient 2 de l'appareil de décharge 4 selon l'invention, mis en oeuvre par le système de détection 1 de la figure 2, va maintenant être décrit en référence à la figure 3.

Au cours d'une étape initiale 64, les moyens 20 du premier dispositif électronique de géolocalisation 10 acquièrent des données de géolocalisation. Cette acquisition est effectuée sur une base périodique, après rebouclage du procédé, que le patient 2 porte ou non l'appareil de décharge 4, et ait initié un mouvement de marche ou non.

Dans l'exemple de réalisation particulier de la figure 2 selon lequel le système de détection 1 comporte un deuxième dispositif de géolocalisation 16, au cours d'une étape parallèle 66, les moyens 32 du deuxième dispositif électronique de géolocalisation 16 acquièrent des données de géolocalisation. Cette acquisition est effectuée sur une base périodique, après rebouclage du procédé, que le patient 2 porte ou non le deuxième dispositif de géolocalisation 16, et ait initié un mouvement de marche ou non.

De préférence, au cours d'une étape 67A suivant l'étape 64, le capteur de mouvement 18 du premier dispositif électronique de géolocalisation 10 détecte le cas échéant un mouvement du premier dispositif 10, indiquant un mouvement du patient 2. A l'issue de cette étape 67A, le capteur de mouvement 18 délivre le cas échéant un signal au microcontrôleur programmable 24, qui lui-même délivre un signal de commande d'émission aux moyens 22 d'émission de données de géolocalisation.

Dans l'exemple de réalisation particulier de la figure 2, au cours d'une étape préférentielle 67B qui suit l'étape 66, le capteur de mouvement 30 du deuxième dispositif électronique de géolocalisation 16 détecte le cas échéant un mouvement du deuxième dispositif 16, indiquant un mouvement du patient 2. A l'issue de cette étape 67B, le capteur de mouvement 30 délivre le cas échéant un signal au microncontrôleur programmable 36, qui lui-même délivre un signal de commande d'émission aux moyens 34 d'émission de données de géolocalisation.

Au cours d'une étape 68 suivant l'étape 64 ou l'étape 67A le cas échéant, les moyens 22 d'émission de données de géolocalisation du premier dispositif de géolocalisation 10 émettent les données de géolocalisation acquises par les moyens 20, sur le réseau de communication 14, à destination de l'équipement de traitement de données 12. Dans l'exemple de réalisation selon lequel le premier dispositif de géolocalisation 10 comporte un capteur de mouvement 18, les moyens d'émission 22 n'émettent les données acquises que si un mouvement a été détecté par le capteur de mouvement 18. Dans l'exemple de réalisation selon lequel le premier dispositif électronique de géolocalisation 10 comprend un podomètre 23, les données émises par les moyens 22 d'émission de données comprennent, outre les données de géolocalisation, les données de mesure du nombre de pas du patient 2 fournies par le podomètre 23.

Dans l'exemple de réalisation particulier de la figure 2, au cours d'une étape 70 suivant l'étape 66 ou l'étape 67B le cas échéant, les moyens 34 d'émission de données de géolocalisation du deuxième dispositif de géolocalisation 16 émettent les données de géolocalisation acquises par les moyens 32, sur le réseau de communication 14, à destination de l'équipement de traitement de données 12. Dans l'exemple de réalisation selon lequel le deuxième dispositif de géolocalisation 16 comporte un capteur de mouvement 30, les moyens d'émission 34 n'émettent les données acquises que si un mouvement a été détecté par le capteur de mouvement 30. Dans l'exemple de réalisation selon lequel le deuxième dispositif électronique de géolocalisation 16 comprend un podomètre 35, les données émises par les moyens 34 d'émission de données comprennent, outre les données de géolocalisation, les données de mesure du nombre de pas du patient 2 fournies par le podomètre 35.

Au cours d'une étape 71 suivant l'étape 68, l'équipement 12 de traitement de données détermine s'il a reçu, de la part du premier dispositif de géolocalisation 10, des données de géolocalisation présentant des valeurs successives distinctes. En variante, dans l'exemple de réalisation selon lequel le premier dispositif de géolocalisation 10 comprend un capteur de mouvement 18, l'équipement 12 de traitement de données détermine s'il a reçu des données de géolocalisation de la part du premier dispositif de géolocalisation 10. Plus précisément, au cours de l'étape 71, l'application 46 est mise en oeuvre par le processeur 42 et effectue cette détermination. Dans tous les cas, si la réponse à cette détermination est non, le procédé passe à une étape suivante 74 décrite par la suite.

Si la réponse à cette détermination est oui, l'équipement 12 de traitement de données détecte, au cours d'une étape suivante 72, le port par le patient 2 de l'appareil 4 de décharge de plaies des pieds. Plus précisément, au cours de l'étape 72, l'application 46, mise en oeuvre par le processeur 42, détecte un mouvement de l'appareil de décharge 4 et en déduit le port par le patient 2 de l'appareil de décharge 4. Avantageusement, l'application 46, une fois mise en oeuvre par le processeur 42, détermine la distance parcourue par le patient 2 muni de l'appareil de décharge 4 ou encore la durée de port de l'appareil de décharge 4. Avantageusement encore, l'application 46, une fois mise en oeuvre par le processeur 42, génère des tableaux et/ou des listes de suivi de l'activité du patient 2, et/ou des données d'actigraphie - actimétrie relatives au patient 2, et/ou des données comparatives de l'activité du patient 2, et/ou des données indicatives de l'état des balises de traçage GPS ou des moyens d'alimentation électrique 26, 38. Ces informations générées périodiquement peuvent avantageusement être visualisées par un utilisateur accédant à l'interface web du serveur 40, via le réseau de communication 14. Pour ce faire, le patient 2 peut télécharger une application spécifique sur un appareil de communication mobile de type smartphone par exemple, via le réseau de communication 14. Une fois cette application spécifique mise en oeuvre sur son appareil de communication mobile, le patient 2 peut alors accéder, via le réseau de communication 14, à l'interface web du serveur 40, lui permettant de visualiser sur son appareil les informations générées périodiquement par l'application 46 du serveur 40.

Dans l'exemple de réalisation particulier de la figure 2, au cours d'une étape finale 74 suivant l'étape 70 et l'étape 71 ou l'étape 72, autrement dit que l'équipement 12 de traitement de données ait détecté ou non le port par le patient 2 de l'appareil 4 de décharge de plaies des pieds, l'équipement 12 calcule un taux de décharge associé au port par le patient 2 de l'appareil de décharge 4. Plus précisément, au cours de l'étape 74, l'application 46 est mise en oeuvre par le processeur 42, et calcule le taux de décharge, suite à la réception des données de géolocalisation émises par le deuxième dispositif électronique de géolocalisation 16 et éventuellement par le premier dispositif électronique de géolocalisation 10.

A l'issue de l'étape finale 74, le procédé reboucle sur les étapes initiales 64, 66.

En variante, les étapes 64, 66, 67A, 67B, 68 et 70 peuvent être intercalées, et non strictement parallèles.

En variante encore, le système de détection 1 ne comporte pas de deuxième dispositif électronique de géolocalisation 16. Dans ce cas, seules les étapes 64, 67A, 68, 71 et 72 sont mises en oeuvre, le procédé rebouclant à l'issue de l'étape 72. Selon cette variante de réalisation, l'équipement 12 de traitement de données ne calcule aucun taux de décharge.

Avantageusement, le serveur 40 de l'équipement 12 de traitement de données peut déclencher une alarme si aucune activité ou peu d'activité est détectée chez le patient 2. En effet, une telle situation peut être le signe d'une perte de connaissance du patient 2. Plus précisément, dans l'exemple de réalisation préférentiel selon lequel le premier dispositif de géolocalisation 10 comporte un capteur de mouvement 18, le serveur 40 peut déclencher une alarme s'il ne reçoit aucune donnée de géolocalisation de la part du premier dispositif de géolocalisation 10 pendant une durée prédéterminée. En variante, dans l'exemple de réalisation selon lequel le dispositif de géolocalisation 10 ne comporte pas de capteur de mouvement, le serveur 40 peut déclencher une alarme si les données de géolocalisation reçues du premier dispositif de géolocalisation 10 pendant une durée prédéterminée présentent des valeurs constantes ou quasi constantes. Cette alarme peut par exemple se présenter sous la forme de l'envoi d'un message d'alarme 50 à la station de surveillance 52 et/ou à un appareil mobile de communication 74 d'une personne déterminée.

En variante encore, lorsque le patient 2 porte un deuxième dispositif de géolocalisation 16, par exemple sous la forme d'un bracelet électronique, la détection d'une non activité du patient 2 entrainant le déclenchement d'une alarme peut correspondre à une non réception de données de la part du premier dispositif de géolocalisation 10 pendant une durée prédéterminée, corrélée à une non réception de données de la part du deuxième dispositif de géolocalisation 16 pendant cette même durée.

Il est à noter que cette alarme peut prendre plusieurs formes. Cela peut prendre la forme d'un affichage sur un écran dans une salle de surveillance et/ou l'envoi d'un message à une personne déterminée, par exemple via un appareil de communication mobile 74 appartenant à cette personne. Cet envoi à une personne déterminée peut être effectué directement par le serveur 40 via l'envoi d'un message 50 sur le réseau de communication 14, ou indirectement via la station de surveillance 52 après avoir reçu au préalable un message d'alarme 50.

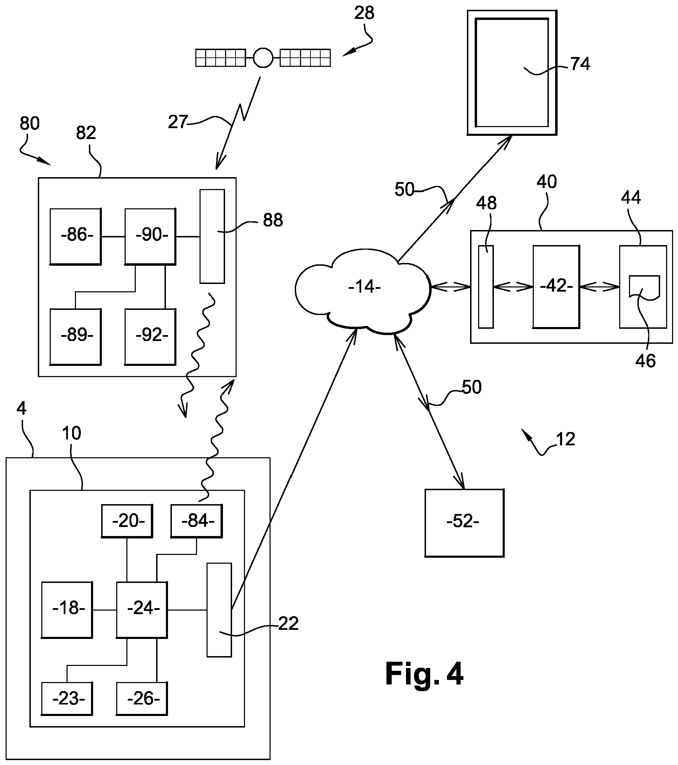

La figure 4 illustre un système 80 de détection du port par un patient 2 d'un appareil 4 de décharge de plaies des pieds, selon un deuxième mode de réalisation de l'invention pour lequel les éléments analogues au premier mode de réalisation, décrit précédemment en regard des figures 1 et 2, sont repérés par des références identiques, et ne sont donc pas décrits à nouveau.

Dans ce deuxième mode de réalisation, le système de détection 80 comprend, outre l'équipement 12 de traitement de données, un dispositif électronique de géolocalisation 10 et un dispositif électronique de communication 82.

Dans ce deuxième mode de réalisation, le dispositif électronique de géolocalisation 10 comporte en outre un émetteur - récepteur 84 de signaux radioélectriques. L'émetteur - récepteur 84 est relié au microcontrôleur programmable 24. L'émetteur - récepteur 84 est par exemple un émetteur - récepteur de signaux radioélectriques à courte distance. L'émetteur - récepteur 84 est par exemple un émetteur - récepteur conforme à la norme Bluetooth.

En outre, dans ce deuxième mode de réalisation, le microcontrôleur programmable 24 est propre à déterminer si une liaison radioélectrique a été établie entre l'émetteur - récepteur 84 et le dispositif électronique de communication 82. Le microcontrôleur 24 est propre à déterminer qu'une liaison radioélectrique a été établie avec le dispositif électronique de communication 82, s'il reçoit au préalable un signal radioélectrique émis par le dispositif électronique de communication 82, suite à l'établissement d'un protocole de communication prédéterminé entre le dispositif de géolocalisation 10 et le dispositif électronique de communication 82. Le microcontrôleur programmable 24 est programmé pour transmettre un signal de commande d'émission de données aux moyens 22 d'émission de données, accompagné d'une première notification si une liaison radioélectrique a été établie entre l'émetteur - récepteur 84 et le dispositif électronique de communication 82 ; et pour transmettre un signal de commande d'émission de données aux moyens 22 d'émission de données, accompagné d'une deuxième notification si aucune liaison radioélectrique n'a été établie avec le dispositif électronique de communication 82.

La première notification est une notification indiquant la présence d'un couplage radioélectrique entre le dispositif de géolocalisation 10 et le dispositif électronique de communication 82, via la liaison radioélectrique établie. La deuxième notification est une notification indiquant l'absence de couplage radioélectrique entre le dispositif de géolocalisation 10 et le dispositif électronique de communication 82.

Le dispositif électronique de communication 82 est adapté pour être fixé directement sur le corps du patient 2, par exemple sur un poignet de ce dernier. Dans ce cas, le dispositif électronique de communication 82 se présente par exemple sous la forme d'un bracelet électronique de communication de signaux radioélectriques. Le bracelet électronique comporte par exemple une balise de communication de signaux radioélectriques à courte distance. Le dispositif électronique de communication 82 présente des dimensions compatibles avec un port sur un membre du patient, typiquement un poignet, qui ne soit pas gênant pour le patient durant son mouvement de marche.

Le dispositif électronique de communication 82 comprend des moyens d'alimentation électrique 92 et un émetteur - récepteur 88 de signaux radioélectriques. De préférence, comme illustré sur la figure 4, le deuxième dispositif électronique de communication 82 comprend en outre un capteur de mouvement 86, un podomètre 89 et un microcontrôleur programmable 90. En variante, le microcontrôleur programmable 90 peut être remplacé par tout type de moyen de calcul et de traitement de données, tel que par exemple un processeur ou un microprocesseur.

Les moyens d'alimentation électrique 92 sont par exemple aptes à fournir une tension électrique nominale sensiblement égale à 12 V. Les moyens d'alimentation électrique 92 sont par exemple formés d'une batterie lithium. Les moyens d'alimentation électrique 92 sont par exemple aptes à fournir une autonomie électrique au dispositif électronique de communication 82 de l'ordre de plusieurs mois.

L'émetteur - récepteur 88 est par exemple un émetteur - récepteur de signaux radioélectriques à courte distance. L'émetteur - récepteur 88 est par exemple un émetteur - récepteur conforme à la norme Bluetooth.

Le capteur de mouvement 86 est relié à l'émetteur - récepteur 88. Dans l'exemple de réalisation de la figure 4, le capteur de mouvement 86 est connecté au microcontrôleur programmable 90, ce dernier étant également connecté à l'émetteur - récepteur 88 de signaux radioélectriques. Le capteur de mouvement 86 est par exemple un accéléromètre linéaire trois axes, apte à délivrer un signal dès qu'une valeur d'accélération non nulle est mesurée. En variante, le capteur de mouvement 86 est un capteur de vitesse instantanée. Dans l'exemple de réalisation selon lequel le dispositif électronique de communication 82 comprend un capteur de mouvement 86, l'émetteur-récepteur 88 est configuré pour émettre des signaux radioélectriques lorsqu'un mouvement du dispositif de communication 82 est détecté par le capteur de mouvement 86. Cet exemple de réalisation permet avantageusement d'économiser de l'énergie électrique au sein des moyens d'alimentation électrique 92, l'émetteur-récepteur 88 n'émettant pas des signaux radioélectriques sur une base périodique mais seulement si un mouvement du dispositif de communication 82 a été détecté par le capteur de mouvement 86.

Le podomètre 89 est connecté au microcontrôleur programmable 90 et est apte à fournir des données de mesure du nombre de pas du patient 2.

Le microcontrôleur programmable 90 est relié au capteur de mouvement 86, à l'émetteur - récepteur 88, au podomètre 89 et aux moyens d'alimentation électrique 92. Le microcontrôleur programmable 90 est par exemple programmé pour transmettre périodiquement un signal de commande d'émission de signaux radioélectriques à l'émetteur - récepteur 88. En variante, dans l'exemple de réalisation selon lequel le dispositif de communication 82 comprend un capteur de mouvement 86, le microcontrôleur programmable 90 est programmé pour transmettre un signal de commande d'émission de signaux radioélectriques à l'émetteur - récepteur 88, lorsqu'un mouvement du dispositif électronique de communication 82 est détecté par le capteur de mouvement 86.

Dans le mode de réalisation particulier de la figure 4 selon lequel le système de détection 80 comprend un dispositif de communication 82, il n'y a plus de transmission de données par un deuxième dispositif de géolocalisation sur le réseau de communication 14. Par conséquent, le système de détection 80 selon le deuxième mode de réalisation de l'invention permet avantageusement de réduire le volume de données de géolocalisation transmises, ainsi que de réduire la consommation de bande passante. En outre, ce système de détection 80 selon le deuxième mode de réalisation permet avantageusement à un praticien suivant le patient 2 de déterminer si le patient porte correctement les éléments de détection du port de l'appareil de décharge, constitués du dispositif de géolocalisation 10 et du dispositif de communication 82. En effet, on se place dans le cas où le dispositif de géolocalisation 10 ne comporte pas de capteur de mouvement, la conclusion étant transposable à l'identique au cas où le dispositif de géolocalisation 10 comporte un capetur de mouvement 18. Si le praticien accède à l'équipement 12 de traitement de données et que ce dernier reçoit, de la part du dispositif électronique de géolocalisation 10, des données de géolocalisation présentant des valeurs successives constantes, plusieurs possibilités sont envisageables :
- le patient 2 porte le dispositif de géolocalisation 10 et le dispositif de communication 82, et est immobile ;
- le patient 2 porte le dispositif de communication 82 mais ne porte pas l'appareil de décharge 4 muni du dispositif de géolocalisation 10, et le patient 2 marche.

Or, l'information d'un couplage radioélectrique ou non entre le dispositif de géolocalisation 10 et le dispositif électronique de communication 82, qui se traduit par la présence d'une première ou d'une deuxième notification accompagnant les données de géolocalisation émises, est reçue par l'équipement 12 de traitement de données. La réception de cette information permet ainsi au praticien accédant à l'équipement 12 de pouvoir discriminer entre ces deux situations et d'ainsi obtenir des informations plus précises quant à l'observance à la décharge de son patient. En outre, la situation selon laquelle le patient 2 porte l'appareil de décharge 4 muni du dispositif de géolocalisation 10 mais ne porte pas le dispositif de communication 82, et selon laquelle le patient 2 marche, peut avantageusement être détectée par l'équipement 12 de traitement de données. En effet, cette situation correspond à une réception de données de géolocalisation présentant des valeurs successives distinctes, accompagnées d'une deuxième notification indicative de l'absence de couplage radioélectrique entre le dispositif de géolocalisation 10 et le dispositif électronique de communication 82. Ainsi, dans ce deuxième mode de réalisation de l'invention, l'équipement 12 de traitement de données est également propre, suite à la réception des données de géolocalisation émises par le dispositif de géolocalisation 10, accompagnées de la première ou la deuxième notification, à calculer le taux de décharge associé au port par le patient 2 de l'appareil de décharge 4. La précision de la détection et de la détermination de la durée de port de l'appareil de décharge par le patient sont ainsi avantageusement améliorées.

En fonctionnement, le procédé de détection du port par le patient 2 de l'appareil de décharge 4 selon l'invention, mis en oeuvre par le système de détection 80 de la figure 4, se déroule comme suit.

Au cours d'une étape initiale, les moyens 20 du dispositif électronique de géolocalisation 10 acquièrent des données de géolocalisation. Cette acquisition est effectuée sur une base périodique, après rebouclage du procédé, que le patient 2 porte ou non l'appareil de décharge 4, et ait initié un mouvement de marche ou non.

De préférence, au cours d'une étape suivante, le capteur de mouvement 18 du dispositif électronique de géolocalisation 10 détecte le cas échéant un mouvement du dispositif 10, indiquant un mouvement du patient 2. A l'issue de cette étape, le capteur de mouvement 18 délivre le cas échéant un signal au microcontrôleur programmable 24, qui lui-même délivre un signal de commande d'émission aux moyens 22 d'émission de données de géolocalisation.

Au cours d'une étape parallèle ou suivante, le microcontrôleur programmable 24 détermine si une liaison radioélectrique a été établie entre l'émetteur - récepteur 84 du dispositif de géolocalisation 10 et l'émetteur - récepteur 88 du dispositif électronique de communication 82. En fonction de cette détermination, le microcontrôleur programmable 24 génère la première ou la deuxième notification.

Au cours d'une étape suivante, les moyens 22 d'émission de données de géolocalisation du dispositif de géolocalisation 10 émettent les données de géolocalisation acquises par les moyens 20 sur le réseau de communication 14, à destination de l'équipement de traitement de données 12, accompagnées de la première ou de la deuxième notification. Dans l'exemple de réalisation selon lequel le dispositif de géolocalisation 10 comporte un capteur de mouvement 18, les moyens d'émission 22 n'émettent les données acquises que si un mouvement a été détecté par le capteur de mouvement 18. Dans l'exemple de réalisation selon lequel le dispositif électronique de géolocalisation 10 et/ou le dispositif électronique de communication 82 comprend un podomètre 23, 89, les données émises par les moyens 22 d'émission de données comprennent, outre les données de géolocalisation, les données de mesure du nombre de pas du patient 2 fournies par le podomètre 23, 89.

Au cours d'une étape suivante, l'équipement 12 de traitement de données détermine s'il a reçu, de la part du dispositif de géolocalisation 10, des données de géolocalisation présentant des valeurs successives distinctes. En variante, dans l'exemple de réalisation selon lequel le dispositif de géolocalisation 10 comprend un capteur de mouvement 18, l'équipement 12 de traitement de données détermine s'il a reçu des données de géolocalisation de la part du dispositif de géolocalisation 10. Plus précisément, au cours de cette étape, l'application 46 est mise en oeuvre par le processeur 42 et effectue cette détermination. Dans tous les cas, si la réponse à cette détermination est non, le procédé passe à une étape finale décrite par la suite.

Si la réponse à cette détermination est oui, l'équipement 12 de traitement de données détecte, au cours d'une étape suivante, le port par le patient 2 de l'appareil 4 de décharge de plaies des pieds. Plus précisément, au cours de cette étape, l'application 46, mise en oeuvre par le processeur 42, détecte un mouvement de l'appareil de décharge 4 et en déduit le port par le patient 2 de l'appareil de décharge 4. Avantageusement, l'application 46, une fois mise en oeuvre par le processeur 42, détermine la distance parcourue par le patient 2 muni de l'appareil de décharge 4 ou encore la durée de port de l'appareil de décharge 4. Avantageusement encore, l'application 46, une fois mise en oeuvre par le processeur 42, génère des tableaux et/ou des listes de suivi de l'activité du patient 2, et/ou des données d'actigraphie - actimétrie relatives au patient 2, et/ou des données comparatives de l'activité du patient 2, et/ou des données indicatives de l'état des balises de traçage GPS ou des moyens d'alimentation électrique 26.

Au cours d'une étape finale suivante, autrement dit que l'équipement 12 de traitement de données ait détecté ou non le port par le patient 2 de l'appareil 4 de décharge de plaies des pieds, l'équipement 12 calcule un taux de décharge associé au port par le patient 2 de l'appareil de décharge 4. Plus précisément, au cours de cette étape finale, l'application 46 est mise en oeuvre par le processeur 42, et calcule le taux de décharge, suite à la réception ou non des données de géolocalisation émises par le dispositif électronique de géolocalisation 10, accompagnées le cas échéant de la première ou de la deuxième notification .

A l'issue de l'étape finale, le procédé reboucle sur l'étape initiale.

Un troisième mode de réalisation d'un système de détection du port par un patient 2 d'un appareil 4 de décharge de plaies des pieds, non représenté sur les figures, peut consister en une combinaison entre les premier et deuxième modes de réalisation représentés sur les figures 2 et 4 respectivement. Plus précisément, selon ce troisième mode de réalisation de l'invention, le système de détection comprend, outre l'équipement 12 de traitement de données, un premier dispositif électronique de géolocalisation 10, un deuxième dispositif électronique de géolocalisation 16 et un dispositif électronique de communication 82. De préférence, le deuxième dispositif électronique de géolocalisation 16 et le dispositif électronique de communication 82 sont agencés au sein d'un même boîtier, typiquement un bracelet électronique dont les dimensions sont compatibles avec un port sur un membre du patient, par exemple un poignet.

Le fonctionnement du système de détection selon ce troisième mode de réalisation étant analogue au fonctionnement du système de détection 1 selon le premier mode de réalisation, combiné au fonctionnement du système de détection 80 selon le deuxième mode de réalisation, celui-ci ne sera pas décrit plus en détail.

Le système de détection du port par un patient d'un appareil de décharge de plaies des pieds selon l'invention offre plusieurs avantages :
- pour fonctionner, le système ne requiert aucune connexion à un réseau local sans fil WLAN (de l'anglais Wireless Local Area Network) chez le patient ;
- le système permet avantageusement un fonctionnement en intérieur aussi bien qu'en extérieur ;
- la transmission à l'équipement de traitement de données des données de géolocalisation par le premier dispositif de géolocalisation, comme par le deuxième dispositif de géolocalisation, s'effectue automatiquement sans aucune action du patient ;
- le système permet avantageusement à un patient de pouvoir visualiser ses données et ses temps de décharge via la connexion au réseau de communication d'un appareil de communication mobile du patient.

L'invention a été décrite en référence à un système de détection du port par un patient d'un appareil de décharge de plaies des pieds. Bien qu'un seul appareil de décharge soit représenté sur les figures 1 et 2, il est entendu que le système de détection s'applique de la même manière si le patient porte deux appareils de décharge, le système selon l'invention permettant de détecter le port de chacun des appareils de décharge. De même, le système de détection selon l'invention s'applique de la même manière à un ensemble de patients portant chacun un ou plusieurs appareils de décharge de plaies des pieds, le système de détection comportant dans ce cas plusieurs dispositifs électroniques de géolocalisation 10, chaque dispositif de géolocalisation 10 étant adapté pour être solidarisé à un appareil de décharge. Dans ce dernier cas, l'équipement de traitement de données du système de détection selon l'invention est propre à générer périodiquement des tableaux et/ou des listes de suivi de plusieurs appareils de décharge, donc de plusieurs patients.

Dans les revendications, le mot « comprenant » n'exclut pas d'autres éléments.

## Revendications

1. Système (1 ; 80) de détection du port par un patient (2) d'un appareil (4) de décharge de plaies des pieds, le système (1) comprenant un dispositif électronique de géolocalisation (10) et un équipement (12) de traitement de données relié au dispositif électronique de géolocalisation (10) via un réseau de communication (14), le dispositif électronique de géolocalisation (10) étant adapté pour être solidarisé à l'appareil de décharge (4) et comportant des moyens d'alimentation électrique (26), des moyens (20) d'acquisition de données de géolocalisation et des moyens (22) d'émission de données de géolocalisation sur le réseau de communication (14) à destination de l'équipement (12) de traitement de données, les moyens (22) d'émission de données étant reliés aux moyens d'acquisition (20), l'équipement (12) de traitement de données étant propre, suite à la réception de données de géolocalisation émises par le dispositif électronique de géolocalisation (10), à détecter un mouvement de l'appareil (4) de décharge de plaies des pieds et à en déduire le port par le patient (2) dudit appareil de décharge (4).

2. Système (1) selon la revendication 1, dans lequel le dispositif électronique de géolocalisation (10) comprend en outre un capteur de mouvement (18) relié aux moyens (22) d'émission de données de géolocalisation, les moyens (22) d'émission de données de géolocalisation étant configurés pour transmettre des données de géolocalisation à l'équipement (12) de traitement de données lorsqu'un mouvement du dispositif (10) est détecté par le capteur de mouvement (18).

3. Système (1) selon la revendication 1 ou 2, comprenant en outre un deuxième dispositif électronique de géolocalisation (16) adapté pour être fixé directement sur le corps du patient (2), le deuxième dispositif de géolocalisation (16) étant relié à l'équipement (12) de traitement de données via le réseau de communication (14) et comprenant des deuxièmes moyens d'alimentation électrique (38), des deuxièmes moyens (32) d'acquisition de données de géolocalisation et des deuxièmes moyens (34) d'émission de données de géolocalisation sur le réseau de communication (14) à destination de l'équipement (12) de traitement de données, les deuxièmes moyens (34) d'émission de données étant reliés aux deuxièmes moyens d'acquisition (32).

4. Système (1) selon la revendication 3, dans lequel le deuxième dispositif électronique de géolocalisation (16) comprend en outre un deuxième capteur de mouvement (30) relié aux deuxièmes moyens (34) d'émission de données de géolocalisation, les deuxièmes moyens (34) d'émission de données de géolocalisation étant configurés pour transmettre des données de géolocalisation à l'équipement (12) de traitement de données lorsqu'un mouvement du dispositif (16) est détecté par le deuxième capteur de mouvement (30).

5. Système (1) selon la revendication 2 ou 4, dans lequel l'un au moins des dispositifs de géolocalisation (10, 16) comprend une balise de traçage GPS à déclenchement instantané sur mouvement du dispositif (10, 16).

6. Système (1) selon la revendication 3 ou 4, ou selon la revendication 5 lorsqu'elle dépend de la revendication 4, dans lequel l'équipement (12) de traitement de données est propre, suite à la réception des données de géolocalisation émises par les dispositifs électroniques de géolocalisation (10, 16), à calculer un taux de décharge associé au port par le patient (2) de l'appareil (4) de décharge de plaies des pieds.

7. Système (80) selon l'une quelconque des revendications précédentes, comprenant en outre un dispositif électronique de communication (82) adapté pour être fixé directement sur le corps du patient (2), le dispositif électronique de communication (82) comprenant des moyens d'alimentation électrique (92) et un émetteur - récepteur (88) de signaux radioélectriques.

8. Système (80) selon la revendication 7, dans lequel le dispositif électronique de communication (82) comprend en outre un capteur de mouvement (86) relié à l'émetteur - récepteur (88) de signaux radioélectriques, l'émetteur - récepteur (88) étant configuré pour émettre des signaux radioélectriques lorsqu'un mouvement du dispositif de communication (82) est détecté par le capteur de mouvement (86).

9. Système (80) selon la revendication 7 ou 8, dans lequel le dispositif électronique de géolocalisation (10) comprend en outre un microcontrôleur programmable (24) relié aux moyens (22) d'émission de données, et un émetteur - récepteur (84) de signaux radioélectriques relié au microcontrôleur programmable (24), le microcontrôleur (24) étant programmé pour transmettre un signal de commande d'émission de données de géolocalisation aux moyens (22) d'émission de données, accompagné d'une première notification si une liaison radioélectrique a été établie entre l'émetteur - récepteur (84) et le dispositif électronique de communication (82) ; et pour transmettre un signal de commande d'émission de données de géolocalisation aux moyens (22) d'émission de données, accompagné d'une deuxième notification, différente de la première notification, si aucune liaison radioélectrique n'a été établie entre l'émetteur - récepteur (84) et le dispositif électronique de communication (82).

10. Système (80) selon la revendication 9, dans lequel l'équipement (12) de traitement de données est propre, suite à la réception des données de géolocalisation émises par le dispositif électronique de géolocalisation (10) et de la première ou de la deuxième notification, à calculer un taux de décharge associé au port par le patient (2) de l'appareil (4) de décharge de plaies des pieds.

11. Système (1 ; 80) selon l'une quelconque des revendications précédentes, dans lequel l'équipement (12) de traitement de données comporte un serveur (40), le serveur (40) comprenant un processeur (42) et une mémoire (44) reliée au processeur (42), la mémoire (44) stockant une application (46), l'application (46) étant propre, lorsqu'elle est mise en oeuvre par le processeur (42), à détecter le mouvement de l'appareil (4) de décharge de plaies des pieds et à en déduire le port par le patient (2) dudit appareil de décharge (4), suite à la réception des données de géolocalisation émises par le dispositif électronique de géolocalisation (10).

12. Procédé de détection du port par un patient (2) d'un appareil (4) de décharge de plaies des pieds, le procédé étant mis en oeuvre par un système de détection (1 ; 80) comprenant un dispositif électronique de géolocalisation (10) et un équipement (12) de traitement de données relié au dispositif électronique de géolocalisation (10) via un réseau de communication (14), le dispositif électronique de géolocalisation (10) étant solidarisé à l'appareil de décharge (4) et comportant des moyens d'alimentation électrique (26), des moyens (20) d'acquisition de données de géolocalisation, et des moyens (22) d'émission de données de géolocalisation sur le réseau de communication (14) à destination de l'équipement de traitement de données (12), les moyens (22) d'émission de données étant reliés aux moyens d'acquisition (20), le procédé comprenant les étapes suivantes :
• acquisition (64) de données de géolocalisation par les moyens (20) d'acquisition de données ;
• émission (68) des données de géolocalisation acquises sur le réseau de communication (14), à destination de l'équipement (12) de traitement de données ;
• détection (71, 72) par l'équipement (12) de traitement de données, à partir des données de géolocalisation reçues, d'un mouvement de l'appareil (4) de décharge de plaies des pieds, et, par suite, du port par le patient (2) dudit appareil de décharge (4).

13. Procédé selon la revendication 12, dans lequel le système de détection (80) comprend en outre un dispositif électronique de communication (82) fixé directement sur le corps du patient, le dispositif électronique de communication (82) comprenant des moyens d'alimentation électrique (92) et un émetteur - récepteur (88) de signaux radioélectriques, le dispositif électronique de géolocalisation (10) comprenant en outre un microcontrôleur programmable (24) relié aux moyens (22) d'émission de données, et un émetteur - récepteur (84) de signaux radioélectriques relié au microcontrôleur programmable (24), le procédé comprenant en outre les étapes suivantes :
• détermination, par le microcontrôleur programmable (24), si une liaison radioélectrique a été établie entre l'émetteur-récepteur (84) du dispositif électronique de géolocalisation (10) et l'émetteur-récepteur (88) du dispositif électronique de communication (82) ;
• transmission, par le microcontrôleur programmable (24), d'un signal de commande d'émission de données de géolocalisation aux moyens (22) d'émission de données, accompagné d'une première notification si une liaison radioélectrique a été établie entre le dispositif électronique de géolocalisation (10) et le dispositif électronique de communication (82) ; ou d'une deuxième notification, différente de la première notification, si aucune liaison radioélectrique n'a été établie entre le dispositif électronique de géolocalisation (10) et le dispositif électronique de communication (82) ;
• calcul, par l'équipement (12) de traitement de données, d'un taux de décharge associé au port par le patient de l'appareil (4) de décharge de plaies des pieds, que l'équipement (12) de traitement de données ait détecté ou non le port par le patient de l'appareil (4) de décharge de plaies des pieds ;
et dans lequel, lors de l'étape (68) d'émission des données de géolocalisation acquises, lesdites données de géolocalisation émises sont accompagnées de la première ou de la deuxième notification.

14. Produit programme d'ordinateur téléchargeable depuis un réseau de communication (14) et/ou enregistré sur un support lisible par ordinateur et/ou exécutable par un processeur (42), **caractérisé en ce qu'**il comprend des instructions de programme, lesdites instructions de programme formant l'application (46) du serveur (40) de l'équipement (12) de traitement de données selon la revendication 11, lesdites instructions de programme étant adaptées pour mettre en oeuvre au moins les étapes (71, 72) de détection d'un mouvement de l'appareil (4) de décharge de plaies des pieds, et, par suite, du port par le patient dudit appareil de décharge (4), du procédé selon la revendication 12 ou 13 lorsque le produit programme est exécuté sur ledit serveur (40).
